# EUROPÄISCHE PATENTSCHRIFT

(11) **EP 2 051 659 B1**
(45) Veröffentlichungstag und Bekanntmachung des Hinweises auf die Patenterteilung: **11.04.2018**
(21) Anmeldenummer: 07801561.7
(22) Anmeldetag: 08.08.2007
(51) Int. Cl.: A61L 27/30, A61C 8/00, C23C 24/08, C23C 26/00, C23C 30/00, A61L 27/56, A61F 2/30

(54) **VERFAHREN ZUM HERSTELLEN EINER PORÖSEN, KERAMISCHEN OBERFLÄCHENSCHICHT**
METHOD FOR THE PRODUCTION OF A POROUS, CERAMIC SURFACE LAYER
PROCÉDÉ POUR LA PRÉPARATION D'UNE COUCHE SUPERFICIELLE CÉRAMIQUE POREUSE

(30) Priorität: 08.08.2006 DE 102006037067
(43) Veröffentlichungstag der Anmeldung: 29.04.2009
(73) Patentinhaber: METOXIT AG, CH-8240 Thayngen (CH)
(72) Erfinder: KÖBEL, Stefan, 8447 Dachsen (CH); WEBER, Wolfram, 78247 Hilzingen (DE); RIEGER, Wolfhart, 8263 Buch (CH)
(74) Vertreter: DTS Zürich
(86) Internationale Anmeldenummer: PCT/EP2007/007023
(87) Internationale Veröffentlichungsnummer: WO 2008/017472

(56) Entgegenhaltungen:
- EP-A- 0 269 745
- EP-A- 0 607 017
- US-A- 4 374 669
- US-A- 4 737 411

## Beschreibung

Die vorliegende Erfindung bezieht sich auf ein Verfahren zum Herstellen einer porösen, keramischen Oberflächenschicht für ein Implantat.
Implantate nehmen in der heutigen Medizin einen immer größeren Stellenwert ein. Es gibt Implantate in den unterschiedlichsten Ausführungsformen, wie medizinische Implantate (Herzschrittmacher, Gelenkimplantate, Dentalimplantat), plastische Implantate (Brustimplantat), funktionelle Implantate mit RFID-Chips.
Prinzipiell werden Implantate vom Körper als Fremdkörper erkannt. Dabei kann der Fall eintreten, dass der Körper ein Implantat einkapselt und es beispielsweise zu keiner mechanisch belastbaren Verbindung zwischen Knochen und Implantat kommt.
Im Stand der Technik wird durch geeignete Wahl des Implantatmaterials erreicht, dass Gewebe, z.B. Knochenzellen, die Implantatoberfläche besiedeln. Dies ist beispielsweise bei Titan-Oberflächen oder auch bei einigen oxidkeramischen Implantatoberflächen der Fall. Die mechanische Belastbarkeit einer dabei vorkommenden Grenzfläche wird wesentlich von ihrer Topographie bestimmt, weshalb Dental-Implantate aus Titan an ihrer Oberfläche strukturiert sind.
Aus Untersuchungen ist bekannt, dass die Poren der Oberfläche eine bestimmte Größe aufweisen müssen, damit eine optimale mechanische Belastbarkeit der Verbindung des Implantats zum Knochen erzielt wird.
Poren in der Oberfläche des Implantats werden gemäß dem Stand der Technik mittels eines Sandstrahl-Verfahrens und eines darauffolgenden Ätzens erhalten.
Die EP1450722 beschreibt ein Dentalimplantat, welches mit einem abtragenden Verfahren auf eine Rautiefe zwischen 4 und 20 Mikrometer aufgeraut wird.
Die DE19858501 beschreibt ein Verfahren zur Bioaktivierung keramischer Implantat-Oberflächen durch die Behandlung mit Lauge.
Weiter beschreibt die WO 2005/02771 ein zweistufiges Verfahren zum Aufbringen einer porösen Schicht auf einer bereits porösen Oberfläche.

Die EP0607017 offenbart ein Verfahren zur Herstellung einer porösen, keramischen Oberflächenschicht auf einem Implantat, das das Aufbringen einer Mischung aus einem Polymer, einem keramischen Material und einem Lösungsmittel auf ein Substrat umfasst. Die aus dem Stand der Technik bekannten Verfahren lassen sich allerdings auf biokeramische Werkstoffe, insbesondere keramische Implantate nur sehr eingeschränkt übertragen.
Aufgabe der vorliegenden Erfindung ist es daher, ein Verfahren zur Verfügung zu stellen, das eine Herstellung einer porösen, keramischen Oberflächenschicht auf einem Implantat erlaubt, wobei mittels des Verfahrens eine vorherdefinierte Porosität der Implantatoberfläche durch Aufbringen einer einzigen Schicht direkt auf dem Substrat erzielt werden kann.
Diese Aufgabe wird durch das erfindungsgemäße Verfahren gemäß Anspruch 1 gelöst.
Weitere vorteilhafte Ausgestaltungen der Erfindung sind in den abhängigen Ansprüchen angegeben.
Gemäß einem ersten Aspekt der vorliegenden Erfindung ist ein Verfahren zum Herstellen einer porösen, keramischen Oberflächenschicht (2) auf einem Substrat (1) vorgesehen, wobei das Verfahren den Schritt eines Aufbringens einer Mischung auf dem Substrat (1) aufweist, wobei die Mischung, unter anderem, umfasst:
mindestens ein Polymer und
mindestens ein keramisches Material.
Unter einer porösen, keramischen Oberflächenschicht versteht man eine Schicht, die eine keramische Zusammensetzung mit vorherdefinierter Porosität und Rauhigkeit aufweist, um damit eine "Verzahnung" von nachwachsendem Gewebe mit einem Implantat zu ermöglichen, womit ein fester Verbund zwischen Gewebe bzw. Knochen und Implantat entsteht. Die poröse, keramische Oberflächenschicht ist bevorzugt biokompatibel, d.h., dass sie optimal mit nachwachsendem Gewebe bzw. Knochen verwächst und somit vom Körper nicht abgestoßen wird.
Bei der vorliegenden Erfindung wird die poröse Oberflächenschicht nach einem Sinterprozess aus einer Mischung erhalten.
Unter einer Mischung versteht man einen Stoff, der aus mindestens zwei Stoffen besteht. Hier besteht die Mischung unter anderem aus mindestens einem keramischen Material und mindestens einem Polymer. Die Mischung wird auf ein Substrat aufgebracht und nach dem Sintern wird eine vorherdefinierte Oberflächenrauhigkeit ausgeformt. Darüber hinaus enthält die Mischung keramisches Material bzw. eine Keramik.
Unter einem Substrat versteht man ein Material mit gewissen Materialeigenschaften, auf welches die Mischung aufgebracht wird. Das Substrat weist vorherdefinierte Materialeigenschaften (Rauhigkeit, Wärmeausdehnungskoeffizient, chemische Zusammensetzung) auf, um eine entsprechende Aufbringung und eine spätere, durch Sinterprozesse erzielte Verbindung mit einer porösen, keramischen Oberflächenschicht zu ermöglichen. Das Substrat umfasst dabei bevorzugt Materialien, wie sie für Implantate eingesetzt werden, bevorzugt ein keramisches Material bzw. eine technische Keramik aus der Gruppe der Oxidkeramiken, wie Al2O3, ZrO2, ZTA, ATZ, MgO, Spinell, Bioglass, Nitridkeramiken, wie Si3N4 und Carbidkeramiken, wie SiC, am meisten bevorzugt ZrO2 vom Typ Y-TZP mit einer Dichte von mindestens 6,00 g/cm³. Bevorzugt weist das Substrat ähnliche Materialeigenschaften (Gitterkonstante, Wärmeausdehnungskoeffizient), wie die poröse, keramische Oberflächenschicht auf.

Die Abkürzung ZTA steht für "zirconia toughened alumina", welches beispielsweise eine Zusammensetzung von 20% ZrO2 + 80% Al2O3 aufweist.
Die Abkürzung ATZ steht für "alumina toughened zirconia", welches beispielsweise eine Zusammensetzung von 80% ZrO2 + 20% Al2O3 aufweist.
Unter einem keramischen Material versteht man keramische Werkstoffe aus der Gruppe die Zirkonoxid einschliesst und für Bereiche der Medizintechnik und allgemeinen Technik eingesetzt wird. Während eines Sintervorgangs kommt es zur Vereinigung der einzelnen Kristallgruppen, die zunächst in Pulverform vorliegen, wodurch sich beispielsweise die hohe Festigkeit von gesintertem, keramischem Material ergibt. Gesintertes keramisches Material wird auch als technische Keramik bezeichnet.
Unter einem Aufbringen der Mischung auf dem Substrat versteht man, dass die Mischung mit der Substratoberfläche in Kontakt gebracht wird, um eine entsprechende Verbindung zwischen beiden Phasen, also dem Substrat und der Mischung, herzustellen.
Bevorzugt kann das Aufbringen in direkter Weise erfolgen. Unter einem direkten Aufbringen versteht man, dass die Mischung unmittelbar auf das Substrat und ohne Zwischenschichten auf das Substrat aufgebracht wird. Die Mischung befindet sich somit in direktem Kontakt mit dem Substrat, wobei die einzelnen Moleküle bzw. Atome des Substrats direkt mit den Molekülen bzw. Atomen der Mischung wechselwirken können und ähnliche Materialeigenschaften, wie beispielsweise Gitterkonstanten für eine optimale Verbindung der späteren porösen, keramischen Oberflächenschicht auf dem Substrat ausgenutzt werden können.

Bei der vorliegenden Erfindung umfasst die Mischung mindestens ein Polymer und mindestens ein keramisches Material, die miteinander derart vermischt sein können, dass die einzelnen Komponenten pro Volumeneinheit ihrer Einwaage entsprechend statistisch nahezu gleichverteilt sind. Ein optimales Mischresultat kann dabei bevorzugt dadurch entstehen, dass das Polymer auf eine vorherdefinierte Temperatur (beispielsweise unter 200°C) erhitzt wird und anschließend mit dem keramischen Material in geeigneter Weise vermischt wird, wobei aus dem Stand der Technik bekannte Mischverfahren herangezogen werden können.

Die Zersetzung der meisten Polymere (Ausnahme Fluor-Basierende Polymere) ist ab 200 °C markant; diese Temperatur hängt vom Sauerstoffpartialdruck ab, so dass Polymere in sauerstoffarmen oder sauerstofffreien Bedingungen (z.B. Vakuum) erst bei höheren Temperaturen instabil werden.

Unter einem Polymer versteht man eine chemische Verbindung, die aus Ketten oder verzweigten Molekülen besteht, die aus gleichen oder gleichartigen Elementen (Monomeren) bestehen.

Das Polymer stammt bevorzugt aus der Gruppe die umfasst: Polysaccharide, Polyvinylalkohole, Wachsemulsionen, PMMA, Cellulosefasern, Polypropylenfasern, Fettalkoholsulfatzubereitungen oder eine Kombination davon.

Polysaccharide, Polyvinylalkohole und Wachsemulsionen sind typische Beispiele für temporäre Bindemittel. Acrylatglas, Cellulosefasern, Polypropylenfasern und Fettalkoholsulfatzubereitungen sind die erfindungsgemäß bevorzugten Porenbildner. Die erfindungsgemäß bevorzugten Porenbildner bilden nach dem Sintervorgang, wie später beschrieben wird, die Poren in der porösen, keramischen Oberflächenschicht, da sie bei dem Sintervorgang verdampfen oder zersetzt werden.

Dabei ist es möglich, dass jeweils nur ein Polymer der Mischung zugefügt wird oder eine Vielzahl unterschiedlicher Polymere der Mischung zugefügt wird.

Durch die Zugabe des Polymers ergibt sich der Vorteil, dass dieses gewisse Materialeigenschaften aufweist, wie beispielsweise die Partikelgröße des Polymers in der Mischung, welche nach den weiteren Verfahrensschritten, wie Trocknen und Sintern, die Porositätseigenschaft der keramischen Oberflächenschicht auf dem Implantat wesentlich beeinflusst.
Das Polymer tritt dabei als ein Porenbildner auf, da bei dem späteren Sinterprozess, bei einer Temperatur von bevorzugt 1400°C, das Polymer in der keramischen Oberflächenschicht zersetzt oder verdampft und eine von elementarem Kohlenstoff freie poröse, keramische Oberflächenschicht auf dem Substrat, insbesondere dem Implantat, entstehen lässt.
Die Sintertemperatur wird vom Material als solchem, sowie von seiner Korngröße beeinflusst. Je feiner das Material, desto niedriger ist die Sintertemperatur. Auch kann durch das gezielte Einbringen von "Sinteradditiven" diese Temperatur gesenkt werden. Für ZrO2 und Al2O3 ist ein Bereich von 1200 bis 1600 °C bevorzugt, insbesondere von 1350 bis 1450 °C bevorzugt.

Gemäß der vorliegenden Erfindung umfasst die Mischung weiter mindestens ein Lösungsmittel.
Als bevorzugtes Lösungsmittel wird H₂O verwendet. Das Lösungsmittel wird deshalb der Mischung hinzugefügt, um das Polymer geeignet mit dem keramischen Material zu mischen und teilweise aufzulösen, wobei das Polymer in einer dispergierenden Form, wie vorhergehend erläutert wurde, bestehen bleibt. Durch das Hinzufügen des Lösungsmittels (Dispergent) zu der Mischung entsteht eine Dispersion. Dies weist den Vorteil auf, dass die Mischung in Form einer Dispersion bei einem Verarbeitungsvorgang in einem Sprüh- oder Tauchvorgang optimal aufgetragen werden kann.

Im Folgenden wird nur mehr der Begriff Dispersion anstelle des Begriffs Mischung verwendet, da der Mischung ein Lösungsmittel hinzugefügt wurde.

Unter einer Dispersion versteht man ein zwei- bzw. mehrphasiges System, in welchem eine zusammenhängende Phase (Dispergent) weitere Phasen (Dispergiermittel, Partikel) enthält.

In der Dispersion liegt das Polymer bevorzugt in einer Emulsion oder Suspension vor, wobei es in dem Dispergenten unlöslich ist, d.h. das Polymer liegt in "kugelförmiger" Form, gewissermaßen als Polymer-Kügelchen bzw. Polymer-Partikel mit einer bestimmten Partikelgröße vor, wobei die einzelnen Polymer-Partikel derart beabstandet sind, dass sie keinen zusammenhängenden Film bilden. In den Zwischenräumen befindet sich somit das keramische Material.

Beim Hinzufügen des Lösungsmittels ist es bevorzugt, dass das Polymer vorher nicht erwärmt wird. Allerdings ist es auch möglich, dieses vorher zu erwärmen und dann mit dem keramischen Material und Lösungsmittel zu vermischen.

Gemäß einem weiteren Aspekt der vorliegenden Erfindung ist es bevorzugt, dass die Dispersion weiter mindestens ein Dispergiermittel umfasst. Unter einem Dispergiermittel versteht man weitere Phasen in einer Dispersion, wobei bei dem erfindungsgemäßen Verfahren bevorzugt alkalifreie Polyelektrolyte, Carbonsäurezubereitungen sowie Alkanolamine verwendet werden. Das Dispergiermittel dient in erster Linie dazu, die Dispersion hydrophiler zu machen und somit mehr Feststoff, also beispielsweise mehr keramisches Material oder anorganischen Binder, wie später noch vorgestellt wird, der Dispersion zufügen zu können, damit diese optimal gemischt ist und sich einzelne Komponenten besser aufgelöst haben.
Es ist bevorzugt, dass für die Verflüssigung ein Verflüssigungsmittel auf der Basis von Polyelektrolyten oder Carbonsäurezubereitungen zur Anwendung kommt. Die Verflüssigung erfolgt über elektrolytische Wechselwirkungen. Über den Kontakt der dissoziierten Ionen des Verflüssigungsmittels mit den Keramikpartikeln in der Dispersion kommt es zum Ausgleich der Ladung, die im wässrigen System an der Oberfläche der Rohstoffteilchen entsteht. Die danach vorliegenden Teilchen können leicht aneinander vorbeigleiten. Weiterhin wirksam bleiben die statischen Ladungen der Rohstoffpartikel, die eine Abstoßung der Teilchen untereinander bewirken. Eine Verringerung der Viskosität ist die Folge. Zur Verflüssigung und Dispergierung von Nichtoxiden wie Siliciumcarbid und Siliciumnitrid werden Rohstoffe auf der Basis von Alkanolaminen eingesetzt. Diese Verflüssigungsmittel wirken pseudokationisch. Über die Anlagerung des Verflüssigungsmittels an die anionische Oberfläche des zu verflüssigenden Rohstoffes entstehen gleichsinnige Ladungen, die eine Abstoßung der Rohstoffteilchen voneinander zur Folge haben. Der Abstand zwischen den Rohstoffteilchen wird vergrößert, wodurch die Viskosität abnimmt. Die Dispersion umfasst weiter mindestens einen anorganischen Binder. Der anorganische Binder stammt aus der Gruppe der Phosphate (z.B. Al-Monophosphat) oder Silikate oder stellt eine Kombination dieser Komponenten dar. Der anorganische Binder bewirkt, dass nach allen Stadien des Verarbeitungsprozesses, also vom Aufbringen der Dispersion auf dem Substrat, bis zum Sinterprozess, die poröse, keramische Oberflächenschicht, also das erfindungsgemäße Endprodukt, eine hohe Festigkeit erlangt. Ebenso bewirkt der anorganische Binder, dass während und nach dem Trocknungsprozess das keramische Material, also bevorzugt ZrO2 vom Typ Y-TZP, besser aneinander haftet. Das keramische Material stammt aus der Gruppe die umfasst: : Y-TZP, ATZ. Y-TZP steht für auf Yttriumoxid stabilisiertes Tetragonales Zirkonoxid (TZP = tetragonal zirconia polycristal). Unstabilisiertes Zirkonoxid ZrO₂ weist bei Raumtemperatur eine monokline Kristallstruktur und bei Temperaturen über 1170°C eine tetragonale Kristallstruktur auf. Da es beim Phasenübergang zwischen tetragonal zu monoklin zu einem Umklappen der Gitterstruktur kommt, was mit einer Volumenzunahme um ca. 3 % verbunden ist, wird reines ZrO₂ mit Yttriumoxid versetzt, um eine spätere Zerstörung beim Sinterprozess zu vermeiden. Y-TZP dient in der erfindungsgemäßen Dispersion als keramisches Material, welches vergleichbare Eigenschaften wie das keramische Material des Implantats aufweist. Y-TZP ist nach dem Sinterprozess eine technische Keramik, deren chemische und physikalische Eigenschaften sich in der Implantations-Chirurgie bisher sehr bewährt haben.
Darüber hinaus ist es bevorzugt, dass die Mischung bzw. Dispersion, die auf das Substrat aufgebracht und anschließend getrocknet und gesintert wird, direkt auf das Substrat, d.h. ohne einer oder weiterer Zwischenschichten, aufgebracht wird. Dadurch ergibt sich der Vorteil, dass Materialeigenschaften des darunterliegenden Substrats, wie beispielsweise Gitterkonstanten, Wärmeausdehnungskoeffizient, etc. optimal ausgenutzt werden können. Somit kommt es einerseits zu einem besseren Halt der porösen, keramischen Oberflächenschicht auf dem Substrat und andererseits zu einer gewünschten Porosität.

Gemäß einem weiteren Aspekt der vorliegenden Erfindung ist es bevorzugt, dass das Verfahren nach dem Schritt des Aufbringens der Dispersion einen Schritt des Trocknens umfasst.

Der Schritt des Trocknens dient bevorzugt dazu, dass die Dispersion, die direkt auf das Substrat aufgebracht wurde, entsprechend nach dem Abtropfen trocknen kann, damit sich nach einem späteren Sinterprozess eine entsprechende poröse und keramische Oberflächenschicht ausbildet. Zum Trocknen wird das Substrat in eine im Stand der Technik bekannte Trocknungsvorrichtung eingebracht und für eine vorherdefinierte Zeit bei einer konstanten oder konstant ansteigenden Temperatur, die nach einer gewissen Zeit einen Maximalwert erreicht, getrocknet.

Gemäß einem weiteren Aspekt der vorliegenden Erfindung ist es bevorzugt, dass der Schritt des Trocknens bei einer Temperatur zwischen 20°C und 70°C, bevorzugt zwischen 40°C und 60°C, am meisten bevorzugt bei 50°C durchgeführt wird. Dadurch ergibt sich der Vorteil, dass das Substrat mit der darauf aufgebrachten Dispersion optimal und den Materialeigenschaften entsprechend getrocknet werden kann.

Das Trocknen dient bei dem erfindungsgemäßen Verfahren dem Entzug des Dispergenten aus der Schicht. Bevorzugt ist es dabei, die Temperatur zu erhöhen und damit die relative Feuchte der Umgebungsluft zu erniedrigen. Erwärmt man auf Temperaturen über dem Verdampfungspunkt des Dispergenten so verdampft dieser. Darüber hinaus kann die Schicht auch durch Unterdruck vom Dispergenten befreit werden, denn die Druckerniedrigung bewirkt eine Senkung der Verdampfungstemperatur.
Bei einem bevorzugten Einbringen der keramischen Komponente in ein erwärmtes, flüssiges Polymer ist folgendes System bevorzugt: eine keramische Komponente, z.B. Y-TZP, wird mit einem Porenbildner, z.B. PMMA, in ein Polymer eingebracht, welches in überkritischem CO2 gut löslich ist. Dieses Polymer wirkt als Dispergent der Keramik und des Porenbildners und kann in einer Druckkammer, welche CO2 unter hohem Druck enthält, ausgewaschen werden. Eine derartige Kombination ist ebenso mit polymeren Dispergenten bevorzugt, welche wasserlöslich sind, und die analog im Wasserbad entfernt werden können.
Derartige Prozesse kennt man in der keramischen Technik als Entbindern. Jedoch wird bei der vorliegenden Erfindung ein anorganischer Binder eingesetzt, welcher auf diese Weise nicht entfernt werden kann.

Gemäß der vorliegenden Erfindung weist das Verfahren einen Sinterprozess auf. Unter einem Sinterprozess versteht man dabei, dass das Substrat mit der darauf befindlichen getrockneten Dispersion in eine entsprechende Sintervorrichtung gebracht wird, wie sie aus dem Stand der Technik für entsprechende Sinterprozesse bekannt ist.
Dabei ist es bevorzugt, dass der Sinterprozess bei einer Temperatur zwischen 1000 °C und 2000 °C, bevorzugt zwischen 1200 °C und 1600 °C, am meisten bevorzugt zwischen 1350 °C und 1450 °C durchgeführt wird. Durch den Sinterprozess werden, wie vorhergehend bereits erläutert wurde, die Polymer-Partikel, die in der jetzt getrockneten Dispersionsschicht auf dem Substrat eingeschlossen sind, bei einer bestimmten Temperatur zersetzt oder verdampft. Die Sintertemperatur wird bevorzugt an die jeweilige Verdampfungstemperatur des Polymers angepasst wird, d.h. die Sintertemperatur liegt bevorzugt über der Zersetzungs- oder Verdampfungstemperatur des Polymers.

Gemäß einem weiteren Aspekt der vorliegenden Erfindung ist es bevorzugt, dass das in der Dispersion vorliegende Polymer eine vorherdefinierte Partikelgröße aufweist, die bevorzugt zwischen 10 Mikrometer (µm) und 100 Mikrometer liegt, besonders bevorzugt zwischen 20 Mikrometer und 50 Mikrometer liegt. Unter der Partikelgröße des Polymers versteht man dabei den äußeren Durchmesser des Polymer-Partikels. Weiter ist es bevorzugt, dass ein vorherbestimmter Prozentsatz der Polymere in der Dispersion eine bestimmte Partikelgröße aufweist, d.h. mit einer bestimmten Verteilung auftritt, die bevorzugt einer Gauß-Verteilung entspricht.

Während des Sintervorgangs zersetzen oder verdampfen die in der verfestigten Dispersion befindlichen Polymer-Kügelchen.

Ab einer bestimmten Temperatur verfestigt sich die Dispersion zu einem festen Gefüge, die Polymer-Kügelchen zersetzen oder verdampfen und es entstehen in der keramischen Schicht Poren, die sich sowohl im Inneren der keramischen Schicht als auch an der Oberfläche befinden.

Es entstehen oberflächennahe Poren, welche die ursprüngliche Geometrie der Polymer-Kügelchen erkennen lassen. Durch die entsprechend gewählte Geometrie und Größe (10 bis 100 Mikrometer) der Kügelchen kann die später durch den Sinterprozess entstehende Porengröße in der Oberflächenschicht gezielt gesteuert werden. Je größer also der Durchmesser der Polymer-Kügelchen ist, desto rauer wird die Oberfläche der porösen, keramischen Oberflächenschicht werden.

Gemäß einem weiteren Aspekt der vorliegenden Erfindung ist es bevorzugt, dass das Aufbringen der Dispersion mittels eines Tauchvorgangs, eines Sprühvorgangs oder einer Kombination davon erfolgt.

Unter einem Tauchvorgang versteht man bevorzugt, dass das Substrat in einen Behälter mit der darin eingebrachten Dispersion vollständig oder teilweise eingetaucht wird, sodass die Dispersion das Substrat vollständig oder teilweise benetzen kann.

Unter einem Sprühvorgang versteht man bevorzugt, dass das Substrat in eine geeignete Vorrichtung eingebracht bzw. aufgebracht wird, welche ein gleichförmiges Besprühen des Substrats mit der Dispersion erlaubt.

Ebenso kann es sich bei dem Aufbringen der Dispersion auf dem Substrat um eine Kombination von Tauch- und Sprühvorgängen handeln.

Sofern es aufgrund der Zusammensetzung der Mischung bzw. Dispersion technisch notwendig ist zusätzlich zu trocknen, da die Schicht beispielsweise nicht unter Umgebungsbedingungen hinreichend schnell trocknet, so erfolgt das Trocknen bevorzugt sofort nach dem Beschichten.

Die entsprechenden Tauch- und Sprühvorgänge erlauben somit die Dispersion auf eine effiziente, sowie kosten- und zeitsparende Weise aufzubringen. Es wird ferner offenbart, dass der Dispersion zusätzlich weitere keramische Komponenten hinzugefügt werden. Diese keramischen Komponenten gehören bevorzugt zur Gruppe die Oxide, Hydroxide, Phosphate und Carbonate umfasst und für eine zusätzliche Funktionalisierung der porösen Schicht sorgt. Dabei kann der Dispersion zusätzlich ein keramisches Material zugefügt werden, welches chemisch mit dem anorganischen Binder und/oder weiteren Komponenten der Dispersion reagiert. Es wird ferner offenbart, dass der Dispersion zusätzlich im Dispergenten lösliche Salze hinzugefügt werden. Diese Salze bilden beim Trocknen und anschließenden Sintern Substanzen, welche die Biokompatibilität der Beschichtung, deren Einheilen oder aber deren Eigenschaften allgemein (ihre Festigkeit, Haftung am Substrat, etc.) fördern. Es wird ferner offenbart, dass das Substrat nach einem heissisostatischen Nachverdichten (HIP) und vor einem re-Oxidationsbrand mit der porösen keramischen Oberflächenschicht beschichtet wird.
Gemäß einem weiteren Aspekt der vorliegenden Erfindung ist es bevorzugt, dass die poröse keramische Oberflächenschicht gemeinsam mit dem Substrat ein Implantat, bevorzugt ein Dentalimplantat bildet. Sowohl durch die Materialeigenschaften des Substrats, wie auch die der porösen, keramischen Oberflächenschicht ergibt sich ein Implantat, welches eine biokompatible Oberflächenschicht aufweist, die ein Verwachsen bzw. eine Verbindung zwischen dem nachwachsendem Gewebe bzw. des Knochens mit dem Implantat erlaubt, wodurch es zu einer optimalen mechanischen Festigkeit zwischen Implantat und Gewebe bzw. dem Knochen kommt.

Bevorzugte Ausführungsformen der Erfindung werden nachfolgend anhand der beigefügten Zeichnung näher erläutert.
Figur 1 zeigt eine graphische Darstellung der auf ein Implantat aufgebrachten Dispersion.
Figur 2 zeigt eine graphische Darstellung der nach dem Trocknungs- und Sinterprozess gebildeten porösen Oberflächenschicht.

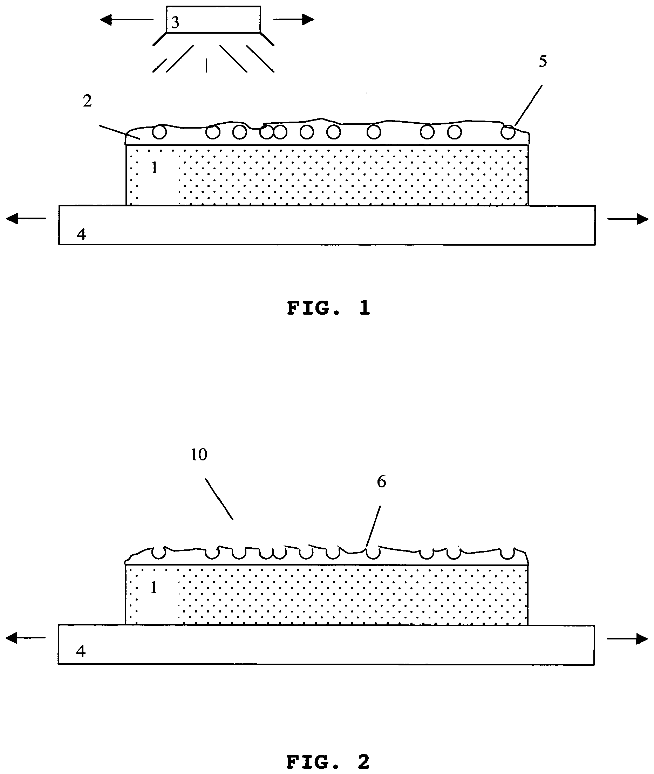

In Figur 1 ist eine graphische Darstellung einer auf einem Substrat 1 aufgebrachten erfindungsgemäßen Dispersion 2 dargestellt, wobei das Aufbringen der Dispersion 2 mittels einer Sprühvorrichtung 3 erfolgt.

Dabei wird in einem ersten Schritt eine erfindungsgemäß bevorzugte Dispersion mit folgenden Komponenten hergestellt:
Von dem keramischen Material Y-TZP werden 300 g von Tosoh TZ-3YS-E, weiter 2,5 g CaCO₃, 90 g BAC-60, welches die Polymer-Kügelchen als spätere Porenbildner nach dem Sinterprozess darstellt, 15 g von Butapur-1 als anorganischer Binder, 3 g Dispex A40 als Dispergent und 529 g H₂O als Lösungsmittel in ein Gefäß gegeben und miteinander vermischt bis eine entsprechend gleichverteilte Dispersion vorliegt. Unter gleichverteilt versteht man, dass die einzelnen Komponenten, die sich in der Dispersion befinden, statistisch gesehen in nahezu gleichen Anteilen in einer Volumeneinheit vorkommen.

Anschließend wird die Dispersion in einen Behälter (nicht dargestellt) einer Sprühvorrichtung 3 eingefüllt, welcher ebenso eine Mischvorrichtung (nicht dargestellt) aufweist, damit die Dispersion ständig gut durchgemischt ist.

Das Substrat 1 wird auf eine Haltevorrichtung 4 gelegt, welches ein keramisches Substrat vom Typ Y-TZP mit der Bezeichnung Metoxit TZP-A ist und eine Dichte von mindestens 6,00 g/cm³ aufweist.

Die in der Graphik dargestellten Pfeile deuten dabei an, dass je nach Anwendungsfall sowohl die Haltevorrichtung 4 als auch die Sprühvorrichtung 3 relativ zu einander bewegt werden können, um ein optimales Auftragungsergebnis der Dispersion 2 auf das Substrat 1 zu erhalten. In diesem Ausführungsbeispiel wurde die Sprühvorrichtung 3 relativ zu der Haltevorrichtung 4 bewegt.

Nachdem 5 ml der Dispersion direkt auf das Substrat 1, d.h. ohne eine weitere Zwischenschicht aufgebracht wurden, hat sich eine flüssige Oberflächenschicht der Disperison 2 auf dem Substrat 1 ausgebildet. Die flüssige Schicht der Dispersion weist dabei eine Stärke von 80 bis 100 Mikrometer auf dem Substrat auf.

In der Figur 1 sind ebenso die in der Dispersion 2 befindlichen Polymer-Kügelchen 5 dargestellt. Sie liegen statistisch verteilt in der Dispersionsschicht auf dem Substrat 1 vor, sowohl im Inneren der Schicht als auch oberflächennah.

Die weiteren in der Dispersion befindlichen Komponenten, wie keramisches Material, anorganischer Binder, Dispergiermittel sind nicht dargestellt.

In Figur 2 ist die poröse Oberflächenschicht nach einem Trocknungsprozess und einem Sinterprozess dargestellt.

Dabei wird zunächst eine Dispersion mit folgender Zusammensetzung hergestellt:
Von dem keramischen Material Y-TZP werden 300 g von Tosoh TZ-3YS-E, weiter 220 g BAC-60 als Porenbildner, 15 g von Butapur-1 als anorganischer Binder, 3 g Dispex A40 als Dispergent und 1200 g H₂O als Lösungsmittel in ein Gefäß gegeben und miteinander vermischt bis eine entsprechend gleichverteilte Dispersion vorliegt. Die bevorzugte Zusammensetzung der Dispersion ist für eine Aufbringung der Dispersion mittels eines Tauchvorganges geeignet, wobei die Dispersion dünnflüssiger als die in Fig. 1 beschriebene Dispersion ist.

Anschließend wird ein Substrat vom Typ Y-TZP mit der Bezeichnung Metoxit TZP-A mit einer Dichte von mindestens 6,00 g/cm³ in den Behälter mit der Dispersion mit seiner vorderen Oberfläche eingetaucht (nicht dargestellt), sodass das Substrat an seiner vorderen Oberfläche mit der Dispersion mit einer Schichtdicke von 80 bis 100 Mikrometer bedeckt ist.

Anschließend wird das Substrat 1 mit der aufgebrachten Dispersion in einer Trockenkammer bei 50°C für 30 bis 60 Minuten getrocknet, bis sich die Dispersion entsprechend verfestigt hat, da Lösungsmittel aus der Dispersion verdampft ist (nicht dargestellt). Dabei wird eine Trockenkammer verwendet wird, wie sie im Stand der Technik üblich ist.

Nach dem Trocknungsprozess wird das Substrat 1 mit der verfestigten Dispersion in eine Sintervorrichtung eingebracht, wobei eine Sintervorrichtung verwendet wird, wie sie im Stand der Technik üblich ist.

Die Sintervorrichtung wird auf eine Temperatur von 1400°C aufgeheizt. Das Substrat mit der verfestigten Dispersion wird dabei für 30 Minuten behandelt.

Dabei verfestigen sich die in der Dispersion vorhandenen Komponenten zu einem porösen, keramischen Gefüge. Es entsteht eine poröse, keramische Oberflächenschicht. Die noch in der verfestigten Dispersion vorhandenen Polymer-Kügelchen, die eine Partikelgröße von 50 Mikrometer aufweisen, sind bei dieser Temperatur bereits zersetzt, da die Zersetzung für dieses Polymer viel tiefer liegt (ca. 200°C).

Dadurch, dass sich die verfestigte Dispersion ab einer bestimmten Temperatur zu einem bestimmten, festen Gefüge verfestigt und die Polymer-Kügelchen zersetzen, entstehen in der keramischen Schicht Poren 6, die sich sowohl im Inneren der keramischen Schicht als auch an der Oberfläche befinden. Dadurch entstehen oberflächliche und oberflächennahe Poren, welche die ursprüngliche Geometrie der Polymer-Kügelchen erkennen lassen, die einzig durch den Sinterprozess entstehen, da die Polymer-Kügelchen bei 1400°C längst zersetzt sind. Somit lässt sich durch die gewählte Geometrie und Größe (10 bis 100 Mikrometer) der Kügelchen die später durch den Sinterprozess entstehende Porengröße in der Oberflächenschicht gezielt steuern. Je größer also der Durchmesser der Polymer-Kügelchen ist, desto rauer wird die Oberfläche der porösen, keramischen Oberflächenschicht.

Weiter ist in Fig. 2 erkennbar, dass die poröse, keramische Oberflächenschicht bedingt durch den Trocknungs- und Sinterprozess eine geringere Dicke als die flüssige Dispersion 2 aufweist. Die Schichtdicke ist dabei auf ungefähr 20 Mikrometer geschrumpft.

Nachfolgend werden zwei Tabellen für bevorzugte Ausführungsbeispiele für Dispersionslösungen angegeben.
Die Tabelle A stellt dabei die Zusammensetzung von drei Beispielen für das Aufbringen mittels Aufsprühen dar, die Tabelle B stellt die Zusammensetzung von drei Beispielen für das Aufbringen mittels eines Tauchvorganges dar.

**Tabelle A**

| **Sprühvorgang (alle Angaben in g)** | | | |
|---|---|---|---|
| **Beispiel** (für BAC-60 anstatt "PAC-60") | | | |
| | **1** | **2** | **3** |
| TZ-3YS-E | 300 | 300 | 300 |
| CaCO₃ | 2,5 | 5 | 15 |
| PAC-60© (Porenbildner) | 90 | 90 | 90 |
| Butapur-1 (anorganischer Binder) | 15 | 15 | 15 |
| Dispex A40 (Dispergent) | 3 | 3 | 3 |
| H₂0 | 529 | 529 | 529 |

**Tabelle B**

| **Tauchvorgang (alle Angaben in g)** | | | |
|---|---|---|---|
| **Beispiel** (für BAC-60 anstatt "PAC-60") | | | |
| | **1** | **2** | **3** |
| TZ-3YS-E | 300 | 300 | 300 |
| CaCO₃ | | | |
| PAC-60© (Porenbildner) | 86 | 132 | 220 |
| Butapur-1 (anorganischer Binder) | 15 | 15 | 15 |
| Dispex A40 (Dispergent) | 3 | 3 | 3 |
| H₂0 | 1200 | 1200 | 1200 |

### Bezugszeichenliste

- 1: Substrat
- 2: Dispersion
- 3: Sprühvorrichtung
- 4: Haltevorrichtung
- 5: Polymer-Kügelchen
- 6: Pore

- 10: poröse Oberflächenschicht

## Patentansprüche

1. Verfahren zum Herstellen einer porösen, keramischen Oberflächenschicht (10) auf einem Substrat (1) für ein Implantat, wobei das Verfahren den Schritt eines Aufbringens einer Mischung auf dem Substrat (1) aufweist, wobei die Mischung umfasst:
mindestens ein Polymer als Porenbildner ;
mindestens ein keramisches Material, das aus der Gruppe stammt, die
umfasst: Y-TZP (Yttriumoxid stabilisiertes Tetragonales Zirkonoxid), ATZ gemäss 80%
ZrO2 und 20% Al2O3; mindestens ein Lösungsmittel; mindestens einen anorganischen Binder aus der Gruppe der Phosphate, Silikate oder aus einer Kombination daraus; und
wobei das Verfahren den Schritt aufweist, dass die poröse Oberflächenschicht nach einem Sinterprozess aus der Mischung erhalten wird.

2. Verfahren gemäß einem der vorhergehenden Ansprüche, wobei die Mischung weiter mindestens einen Dispergenten umfasst.

3. Verfahren gemäß einem der vorhergehenden Ansprüche, wobei das Verfahren nach dem Schritt des Aufbringens der Mischung (2) einen Schritt des Trocknens umfasst.

4. Verfahren gemäß Anspruch 3, wobei der Schritt des Trocknens bei einer Temperatur zwischen 20 °C und 140 °C, bevorzugt zwischen 40 °C und 120 °C, am meisten bevorzugt bei 50 °C durchgeführt wird.

5. Verfahren nach einem der vorhergehenden Ansprüche, wobei der Sinterprozess bei einer Temperatur zwischen 1000°C und 2000°C, insbesondere zwischen 1200°C und 1600°C, insbesondere zwischen 1350°C und 1450°C durchgeführt wird.

6. Verfahren gemäß einem der vorhergehenden Ansprüche, wobei das Substrat aus der Gruppe stammt die umfasst: Oxidkeramik, Al2O3, ZrO2, ZTA, ATZ, MgO, Spinell, Bioglass, Nitridkeramik, Si3N4, Carbidkeramik, SiC, sowie sonstige, nicht notwendigerweise biokompatible, keramische Materialien.

7. Verfahren gemäß einem der Ansprüche 2 bis 6, wobei ein Polymer als Dispergent dient.

8. Verfahren gemäß einem der vorhergehenden Ansprüche, wobei das in der Mischung vorliegende Polymer eine vorherdefinierte Partikelgröße aufweist, die zwischen 10 µm bis 100 µm liegt.

9. Verfahren gemäß einem der vorhergehenden Ansprüche, wobei das Polymer aus der Gruppe stammt, die umfasst: Poly-Vinylacetat, Cellulose, Polysaccharide, Polyvinylalkohole, Wachsemulsionen, Acrylatglas, Cellulosefasern, Polypropylenfasern, Fettalkoholsulfatzubereitungen oder eine Kombination davon.

10. Verfahren gemäß einem der vorhergehenden Ansprüche, wobei das Lösungsmittel H₂0 sowie organische Lösungsmittel mit hydrophilen funktionellen Gruppen umfasst.

11. Verfahren gemäß einem der vorhergehenden Ansprüche, wobei das Aufbringen der Mischung mittels eines Tauchvorgangs, eines Sprühvorgangs oder einer Kombination davon erfolgt.

12. Implantat, umfassend ein Substrat (1) und eine poröse, keramische Oberflächenschicht (10) gemäß einem der vorhergehenden Ansprüche.

## Claims

1. Method for producing a porous, ceramic surface layer (10) on a substrate (1) for an implant, wherein the method has the step of applying a mixture to the substrate (1), wherein the mixture comprises:
at least one polymer as pore forming material;
at least one ceramic material which comes from the group which comprises:
Y-TZP (yttria-stabilized tetragonal zirconia), ATZ corresponding to 80% ZrO2 and 20% Al2O3;
at least one solvent;
at least one inorganic binder from the group of phosphates and silicates or from a combination thereof; and
wherein the method has the step in that the porous surface layer is obtained from the mixture after a sintering process.

2. Method according to one of the preceding claims, wherein the mixture further comprises at least one dispersant.

3. Method according to one of the preceding claims, wherein the method comprises a drying step after the step of applying the mixture (2).

4. Method according to claim 3, wherein the drying step is carried out at a temperature between 20°C and 140°C, preferably between 40°C and 120°C, most preferably at 50°C.

5. Method according to one of the preceding claims, wherein the sintering process is carried out at a temperature between 1000°C and 2000°C, in particular between 1200°C and 1600°C, in particular between 1350°C and 1450°C.

6. Method according to one of the preceding claims, wherein the substrate comes from the group which comprises: oxide ceramic, Al2O3, ZrO2, ZTA, ATZ, MgO, spinel, bioglass, nitride ceramic, Si3N4, carbide ceramic, SiC, as well as other, not necessarily biocompatible, ceramic materials.

7. Method according to one of claims 2 to 6, wherein a polymer is used as dispersant.

8. Method according to one of the preceding claims, wherein the polymer present in the mixture has a predefined particle size, which is between 10 µm and 100 µm.

9. Method according to one of the preceding claims, wherein the polymer comes from the group which comprises: polyvinyl acetate, cellulose, polysaccharides, polyvinyl alcohols, wax emulsions, acrylic glass, cellulose fibres, polypropylene fibres, fatty alcohol sulphate preparations or a combination thereof.

10. Method according to one of the preceding claims, wherein the solvent comprises H₂O as well as organic solvents with hydrophilic functional groups.

11. Method according to one of the preceding claims, wherein the application of the mixture is effected by means of a dipping process, a spraying process or a combination thereof.

12. Implant, comprising a substrate (1) and a porous, ceramic surface layer (10) according to one of the preceding claims.

## Revendications

1. Procédé de fabrication d'une couche de surface (10) céramique poreuse sur un substrat (1) pour un implant, le procédé comprenant l'étape d'application d'un mélange sur le substrat (1), le mélange comprenant :
au moins un polymère prenant la forme d'un agent porogène ;
au moins une matière céramique provenant du groupe comprenant :
le Y-TZP (oxyde de zirconium tétragonal stabilisé à l'oxyde d'yttrium), l'ATZ fabriqué avec 80 % de ZrO2 et 20 % d'Al2O3 ;
au moins un solvant ;
au moins un liant anorganique appartenant au groupe des phosphates, silicates ou d'une combinaison de ceux-ci ; et
le procédé comprenant l'étape d'obtention de la couche de surface poreuse après un processus de frittage à partir du mélange.

2. Procédé selon l'une quelconque des revendications précédentes, le mélange comprenant en sus au moins un agent de dispersion.

3. Procédé selon l'une quelconque des revendications précédentes, le procédé comprenant une étape de séchage après l'étape d'application du mélange (2).

4. Procédé selon la revendication 3, l'étape de séchage étant réalisée à une température comprise entre 20 °C et 140 °C, de façon préférée entre 40 °C et 120 °C, de façon totalement préférée de 50 °C.

5. Procédé selon l'une quelconque des revendications précédentes, le processus de frittage étant réalisé à une température comprise entre 1000 °C et 2000 °C, notamment entre 1200 °C et 1600 °C, notamment entre 1350 °C et 1450 °C.

6. Procédé selon l'une quelconque des revendications précédentes, le substrat provenant du groupe comprenant : la céramique oxydée, Al2O3, ZrO2, ZTA, ATZ, MgO, le spinelle, le verre bioactif, la céramique nitrurée, Si3N4, la céramique carburée, SiC, ainsi que toutes autres matières céramiques non nécessairement biocompatibles.

7. Procédé selon l'une quelconque des revendications 2 à 6, un polymère servant d'agent de dispersion.

8. Procédé selon l'une quelconque des revendications précédentes, le polymère présent dans le mélange comportant une taille de particules prédéfinie comprise entre 10 µm à 100 µm.

9. Procédé selon l'une quelconque des revendications précédentes, le polymère provenant du groupe comprenant : le polyacétate de vinyle, la cellulose, le polysaccharide, les alcools polyvinyliques, les émulsions de cire, le verre en acrylate, les fibres de cellulose, les fibres de polypropylène, les préparations de sulfate d'alcool gras ou une combinaison de ceux-ci.

10. Procédé selon l'une quelconque des revendications précédentes, le solvant comprend H₂O ainsi que le solvant organique avec des groupes fonctionnels hydrophiles.

11. Procédé selon l'une quelconque des revendications précédentes, l'application du mélange s'effectuant par immersion, pulvérisation ou une combinaison de celles-ci.

12. Implant, comprenant un substrat (1) et une couche de surface (10) céramique poreuse selon l'une quelconque des revendications précédentes.
